# EUROPEAN PATENT SPECIFICATION

(11) **EP 3 190 258 B1**
(45) Date of publication and mention of the grant of the patent: **30.12.2020**
(21) Application number: 15810687.2
(22) Date of filing: 26.06.2015
(51) Int. Cl.: E21B 47/00, G01V 1/40, G01V 3/18, G01N 27/02, G01N 33/24

(54) **METHOD FOR CALCULATING ROCK WATER SATURATION**
VERFAHREN ZUR BERECHNUNG DER WASSERSÄTTIGUNG VON GESTEIN
PROCÉDÉ POUR LE CALCUL DE LA SATURATION EN EAU D'UNE ROCHE

(43) Date of publication of application: 12.07.2017
(73) Proprietor: Southwest Petroleum University, Sichuan 610500 (CN)
(72) Inventor: LIU, Hongqi, Chengdu, Sichuan 610512 (CN); TIAN, Jie, Chengdu, Sichuan 610512 (CN); DENG, Youming, Chengdu, Sichuan 610512 (CN); QIU, Chunning, Chengdu, Sichuan 610512 (CN)
(74) Representative: Sun, Yiming
(86) International application number: PCT/CN2015/082499
(87) International publication number: WO 2016/206091

(56) References cited:
- CN-A- 101 109 726
- CN-A- 102 565 858
- CN-A- 103 527 172
- CN-A- 104 122 593
- US-A- 5 048 328
- ROSEMARY J. KNIGHT ET AL: "The dielectric constant of sandstones, 60 kHz to 4 MHz", GEOPHYSICS, vol. 52, no. 5, May 1987 (1987-05), pages 644-654, XP055419437, US ISSN: 0016-8033, DOI: 10.1190/1.1442332
- KEZHEN HU ET AL: "Theoretical Study of the Dielectric Constant in Porous Sandstone Saturated with Hydrocarbon and Water", IEEE TRANSACTIONS ON GEOSCIENCE AND REMOTE SENSING, IEEE SERVICE CENTER, PISCATAWAY, NJ, US, vol. 38, no. 3, May 2000 (2000-05), XP011021532, ISSN: 0196-2892
- Ben K Sternberg ET AL: "Electrical parameters of soils in the frequency range from 1 kHz to 1 GHz, using lumped-circuit methods", Radio Science, August 2001 (2001-08), pages 709-719, XP055419377, Retrieved from the Internet: URL:http://onlinelibrary.wiley.com/doi/10. 1029/1999RS002232/pdf [retrieved on 2017-10-26]
- Rosemary Jane Knight: "The Dielectric Constant of Sandstones, 5 Hz To 13 MHz", , 1984, XP055419424, Retrieved from the Internet: URL:https://pangea.stanford.edu/department s/geophysics/dropbox/SRB/public/docs/these s/SRB_024_NOV84_Knight.pdf [retrieved on 2017-10-26]

## Description

### Field of the Invention

The invention relates to a calculation method of rock water saturation.

### Description of the Related Art

Most materials in nature are dielectrics. The conduction effect of current of macro materials under the action of an applied electric field is measured by conductivity. Macro materials can be classified by conductivity as follows: the macro materials are called conductors when σ>10⁵Ω⁻¹•cm⁻¹, the macro materials are called insulators when σ<10⁻¹⁰Ω⁻¹•cm⁻¹, the macro materials are called semi-conductors when 10⁻¹⁰<σ<10⁵Ω⁻¹•cm⁻¹. The electric polarization effect of macro materials under the action of an electric field can be measured by dielectric constant (permittivity) ε. Vacuum dielectric constant is equal to 1. In general, the dielectric constant of all materials is larger than 1, that is, ε≥1, and materials with ε≠1 are called dielectrics.

Rocks in the formation have both conductive properties and dielectric properties. The formation can be regarded as a giant dielectric, and the conduction is completed by formation aqueous solution in pores, and the conduction of the aqueous solution is attributed to various ions dissolved therein. In the formation, such ions generally include Na⁺, Mg²⁺, Ca²⁺, K⁺, Cl⁻, OH⁻, HCO₃⁻, SO₄²⁻, CO₃²⁻ and others, which form a conductive path of the formation. Therefore, the electric properties of rocks consist of two parts, one part consists of conductive properties of a path formed by aqueous solution communicated among pores, and the other part consists of dielectric properties presented by non-conductive materials and particles such as mineral particles of rocks, oil and gas molecules and water molecules. But in the formation, the current path whether conductive or dielectric will be affected by the pore geometry of rocks, the relationship between conductive and dielectric properties and pore structure of rocks has been described in lots of literatures in details, and will not be repeated here. Studies on the electric properties of rocks are mainly focused on conductive properties. In 1941, K.S.Cole and R.H.Cole established a dielectric constant Cole-Cole model, thereafter, a large number of scholars began to study the ionic conduction and polarization of rocks so as to analyze the characteristics of dielectric constant of heterogeneous porous media.

In "The dielectric constant of sandstones, 60 kHz to 4 MHz" (XP055419437, DOI: 10.1190/1.1442332) a method to measure rock water saturation is disclosed, where however degassed and deionized water is employed, requiring a long desalination process.

In traditional methods for calculating water saturation, due to the existence of multiple parameters that need to be determined by experimental measurements, on the one hand, the testing expenses of the multiple parameters are relatively expensive, on the other hand, as the number of parameters subject to experimental measurements is large, and many sources of experimental errors exist, it is difficult to guarantee the calculation accuracy of saturation. In addition, for tight oil and gas reservoirs, the reservoir space is a nanoscale pore system, the conductive path is very narrow, and the resistivity is very large. In such case, the conductivity of rocks is getting weak, and dielectric gradually takes the dominant place. Therefore, the electric properties of rocks will no longer conform to traditional conductive models, and the resistivity is less sensitive to water content in rocks.

### Summary of the Invention

To this end, the inventor concluded that permittivity parameter should be adopted to reflect the changes in rock water content through lots of researches and experiments, and put forward a method for calculating water saturation by rock permittivity dispersion. As the dispersion characteristics of permittivity is more significant than those of resistivity, use of the permittivity dispersion of cores to calculate rock water saturation is more suitable for tight reservoirs, and more accurate compared with calculating rock water saturation by resistivity methods.

The present invention is related to a calculation method of rock water saturation described in claim 1.

Core resistivity and permittivity parameters are measured under different saturations in the range of 1Hz-5MHz, the measuring frequency and core resistance-capacitance parameters are automatically recorded by an instrument, then the resistance-capacitance parameters and frequency are fitted in the Excel software by the method established in the three equations above to obtain dispersion index Dc of permittivity, calculate the dispersion D_{fc} according to the formula (2), and establish a relationship as shown in the formula (1) by fitting core saturation with dispersion parameter, then a calculation formula based on the permittivity dispersion index is established, thus accurately calculating rock water saturation, helping further study rocks, and finding new applications and breakthrough in electrical well logging.

### Brief Description of the Drawings

To explain the technical scheme in the embodiments of the invention or in the prior art more clearly, figures required for description of the embodiments or prior art will be introduced briefly. Obviously, figures in the description below are only for embodiments of the invention, and other figures can be obtained by those of ordinary skill in the art without creative work based on the figures.
Fig. 1 is a cross plot of the permittivity of different core saturations; and
Fig. 2 is a cross plot of dispersion and saturation.

### Description of the Preferred Embodiments

The technical scheme in the embodiments of the invention will be described in detail. A calculation equation of water saturation is as follows: $Sw = {aD}_{fc} + b$ (1) in the formula (1):
Sw is water saturation, %;
a is coefficient, (m·Hz)/F;
b is regression parameter, nondimensional;
D_{fc} is permittivity dispersion, F/(m·Hz);
D_{fc} is calculated by the following formula: ${D}_{fc} = \left|\frac{{dC}_{p}}{df}\right| = \left|{C}_{0}\right| {D}_{c} {f}^{- \left(Dc+1\right)}$ (2) in the formula (2):
   Cp-permittivity, F/m;
   *D_{C}*-permittivity dispersion index, nondimensional;
   *f*-frequency, Hz;
   C₀-permittivity coefficient, F;
   Cp, C₀ and Dc are related by the following formula (3): $Cp = {C}_{0} {f}^{- Dc}$ (3)

The calculation method comprises the following steps:
(1) measuring core geometry and weight, and recording in Table 1, specifically:
measuring basic core data:
measuring basic core data including core geometry and dry weight, as shown in Table 1;

**Table 1 Basic parameters of core sample**

| **No.** | **Height/ cm** | Diameter/ cm | Medium pore diameter/c m | Dry weight/ g | Volum e/ cm³ | Porosity/ % | Permeability/ mD |
|---|---|---|---|---|---|---|---|
| 16# | 15 | 10.47 | 1.43 | 3048 | 1267.5 5 | 7.5 | 0.43 |

(2) measuring rock water saturation by displacing saturation, completely saturating the rock with saline solution of a certain concentration generally, with the first saturation Sw=100%;
(3) measuring the resistivity/permittivity parameter of the rock in a frequency band (1Hz-10kHz) in case of Sw=100%, and saving relevant data in an Excel file as shown in Table 2;
(4) changing the rock water saturation by displacing saturation, reducing the rock water saturation (assuming Sw=80%) generally, and then measuring the resistivity/permittivity parameter of the rock in the same frequency band after displacement;
the step (3) and the step (4) are specifically as follows:
carrying out displacing saturation on the core by a high pressure vacuum saturation meter. For the first time, the core is completely saturated, that is, Sw is almost 100%, due to core permeability, it is impossible for all pores to be filled with fluid;
measuring resistivity and permittivity parameters after the first complete saturation (Sw1) by a bridge analyzer, and recording the parameters in Table 3;
carrying out subordinate displacement on the core by a high pressure vacuum saturation meter, calculating saturation Sw2, measuring core resistivity and permittivity parameters after the subordinate displacement, and recording the parameters in Table 3;
repeating the operation until the core cannot be displaced again, then measuring the core resistivity and permittivity in minimum saturation, and recording the core resistivity and permittivity in Table 3; displacing saturation is carried out five times in the embodiment, and all data are recorded in Table 3;

The calculation results of saturation of the core after displacing saturation for five times are listed in Table 2.

**Table 2. Saturation parameters of core after displacing saturation for five times**

| | | | | | | |
|---|---|---|---|---|---|---|
| **16#** | Core weight/g | 3172 | 3128 | 3108 | 3094 | 3050 |
| | **Saturation** | Sw1 | Sw2 | Sw3 | Sw4 | Sw5 |
| | Saturation/% | 88.36 | 56.49 | 42.01 | 31.87 | 0.00 |

**Table 3 Datasheet of capacitance-resistance parameters under different saturations**

| **Resistance-capacitance parameter** | **F/Hz** | **Sw1** | **Sw2** | **Sw3** | **Sw4** | **Sw5** |
|---|---|---|---|---|---|---|
| Resistivity/Ω·m | 24 | 8.38E+02 | 1.04E+03 | 1.61 E+03 | 2.15E+03 | 6.85E+03 |
| | 35 | 8.32E+02 | 1.03E+03 | 1.60E+03 | 2.14E+03 | 6.82E+03 |
| | 192 | 8.07E+02 | 1.00E+03 | 1.57E+03 | 2.11 E+03 | 6.73E+03 |
| | 280 | 8.02E+02 | 9.99E+02 | 1.56E+03 | 2.10E+03 | 6.70E+03 |
| Permittivity//F/m | 24 | 3.82E-07 | 2.46E-07 | 1.33E-07 | 8.12E-08 | 2.15E-08 |
| | 35 | 2.43E-07 | 1.59E-07 | 8.50E-08 | 5.20E-08 | 1.39E-08 |
| | 192 | 3.45E-08 | 2.42E-08 | 1.31 E-08 | 8.56E-09 | 2.32E-09 |
| | 280 | 2.29E-08 | 1.63E-08 | 9.06E-09 | 5.93E-09 | 1.67E-09 |

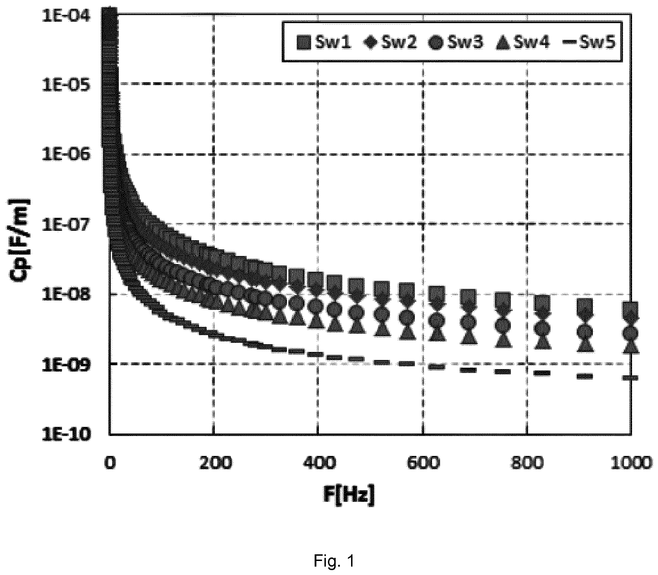

(5) Establishing a Cp-f cross plot by fitting in the Excel file (as shown in Fig. 1) to obtain a permittivity-frequency index relation equation as shown in the formula (3) by fitting,
wherein the frequency index in the formula is Dc parameter; specifically,
establishing cross plots and relation equations of saturations and corresponding permittivity by fitting, as shown in Fig. 1, equations established in the embodiment are as follows:
Sw1=88.36%: Cp1=2×10⁻⁵×F^{-1.239};
Sw2=59.49%: Cp2=1×10⁻⁵×F^{-1.237};
Sw3=42.01%: Cp3=8×10⁻⁶×F^{-1.233};
Sw4=31.87%: Cp4=5×10⁻⁶×F^{-1.224};
Sw5=0 %: Cp5=1×10⁻⁶×F^{-1.192}.

The permittivity dispersion indexes of the corresponding 5 saturations obtained by fitting are:
Dc1=1.239, Dc2=1.237, Dc3=1.233, Dc4=1.224, Dc5=1.192, respectively.
(6) calculating dispersion D_{fc} according to the formula (2); specifically,
deriving saturations and the corresponding permittivity equation to obtain the permittivity dispersion under different saturations, as shown in Tab. 4,

**Table 4 Datasheet of permittivity dispersion under different saturations**

| **Saturation** | **F/Hz** | **Sw1** | **Sw2** | **Sw3** | **Sw4** | **Sw5** |
|---|---|---|---|---|---|---|
| **Dispersion** | | D_{fc}**1** | D_{fc}**2** | D_{fc}**3** | D_{fc}**4** | D_{fc}**5** |
| D_{fc}/F/m/Hz | 24 | 2.28E-08 | 1.44E-08 | 8.50E-09 | 4.99E-09 | 1.92E-09 |
| | 35 | 9.78E-09 | 6.27E-09 | 3.66E-09 | 2.14E-09 | 8.26E-10 |
| | 192 | 2.16E-10 | 1.46E-10 | 8.18E-11 | 4.69E-11 | 1.83E-11 |
| | 280 | 9.29E-11 | 6.37E-11 | 3.52E-11 | 2.01E-11 | 7.89E-12 |

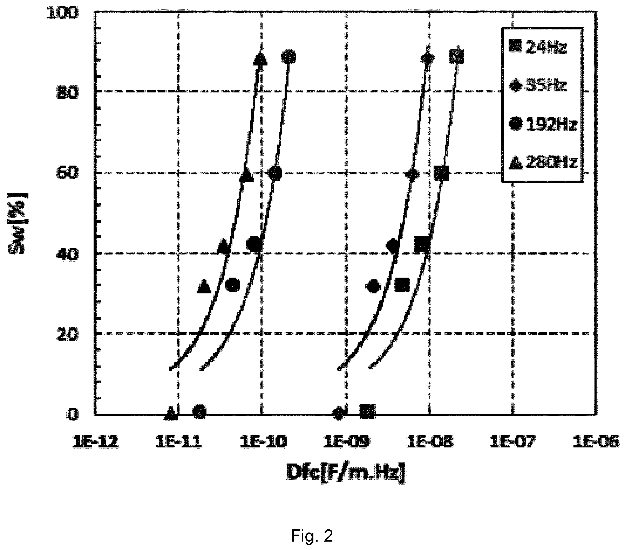

(7) saving core saturation and the calculated dispersion in the Excel file, and establishing an Sw-D_{fc} cross plot (as shown in Table 3) by fitting to obtain a water saturation-dispersion relation equation as shown in the formula (1) by fitting, specifically, drawing a cross plot of different saturations and permittivity dispersion, as shown in Fig. 2, then fitting the data to obtain the following fitting formula of dispersion and saturation:
24Hz: Sw=3.85x109 ×D_{fc} +3.92
35Hz: Sw=8.93x109 ×D_{fc} +3.88
192Hz: Sw=3.98×1011×D_{fc} +3.78
280Hz: Sw=9.23×1011×D_{fc}+3.78
thus establishing a specific formula based on permittivity dispersion index, and accurately calculating the rock water saturation.

## Claims

1. A calculation method of rock water saturation, **characterized in that** the said rock water saturation is calculated by formula (1): Sw=aD_{fc}+b;
in the formula (1):
Sw is water saturation, %;
a is coefficient, (m·Hz)/F;
b is regression parameter, nondimensional;
D_{fc} is permittivity dispersion, F/(m·Hz);
D_{fc} is calculated by formula (2); ${D}_{fc} = \left|\frac{{dC}_{p}}{df}\right| = \left|{C}_{0}\right| {D}_{c} {f}^{- \left(Dc+1\right)}$
in the formula (2):
Cp-permittivity, F/m;
D_{c}-permittivity dispersion index, nondimensional;
f-frequency, Hz;
C₀-permittivity coefficient, F;
Cp, C₀ and D_{c} are related by formula (3): $Cp = {C}_{0} {f}^{- Dc}$ comprising the following steps:
(1) measuring the geometric diameter and weight of the core;
(2) measuring rock water saturation by displacing saturation;
(3a) completely saturating the rock with saline solution of a certain concentration, with the first saturation Sw=100%;
(3b) measuring the permittivity parameter of the rock in a frequency band (1Hz-10kHz) in case of Sw=100%, and saving relevant data;
(4) changing the rock water saturation by displacing saturation, reducing the rock water saturation, and then measuring the permittivity parameter of the rock in the same frequency band after displacement;
(5) establishing a Cp-f cross plot by fitting to obtain a permittivity-frequency index relation equation as shown in the formula (3) by fitting, wherein the fitting parameters in the formula (3) are C₀ and D_{c};
(6) calculating dispersion D_{fc} for each saturation level according to the formula (2); and
(7) saving core saturation and the calculated dispersion data, and establishing an Sw-D_{fc} cross plot by fitting said saved values to obtain a water saturation-dispersion relation equation according to the formula (1).

## Patentansprüche

1. Verfahren zur Berechnung der Wassersättigung von Gestein, **dadurch gekennzeichnet, dass** die Wassersättigung von Gestein mit der Formel (1): Sw=aDfc+b berechnet wird;
in der Formel (1):
S_{w} ist die Wassersättigung, %;
a ist Koeffizient, (m Hz)/F;
b ist Regressionsparameter, dimensionslos;
D_{fc} ist die Permittivitätsdispersion, F/(m·Hz);
D_{fc} wird mit Formel (2) berechnet; ${D}_{fc} = \left|\frac{{dC}_{p}}{df}\right| = \left|{C}_{0}\right| {D}_{c} {f}^{- \left(Dc+1\right)}$ in der Formel (2):
Cp--Permittivität, F/m;
D_{c}-Permittivitätsdispersionsindex, dimensionslos; f--Frequenz, Hz;
C₀-Permittivitätskoeffizient, F;
Cp, C₀ und D_{c} werden durch Formel (3) in Beziehung gesetzt: $Cp = {C}_{0} {f}^{- Dc}$ umfassend die folgenden Schritte:
(1) Messen des geometrischen Durchmessers und Gewichts des Kerns;
(2) Messen der Wassersättigung von Gestein durch Sättigungsverschiebung;
(3a) vollständiges Sättigen des Gesteins mit Kochsalzlösung einer bestimmten Konzentration, mit der ersten Sättigung Sw=100%;
(3b) Messen des Permittivitätsparameters des Gesteins in einem Frequenzband (1 Hz-10 kHz) im Fall von Sw= 100%, und Speichern der relevanten Daten;
(4) Verändern der Wassersättigung des Gesteins durch Sättigungsverschiebung, Reduzieren der Wassersättigung des Gesteins und dann Messen des Permittivitätsparameters des Gesteins im selben Frequenzband nach der Verschiebung;
(5) Erstellen eines Cp-f Kreuzdiagramms durch Anpassung zum Erhalten einer Permittivitäts-Frequenz-Index-Beziehungsgleichung, wie in Formel (3) gezeigt, durch Anpassung, wobei die Anpassungsparameter in Formel (3) C₀ und D_{c} sind;
(6) Berechnen der Dispersion Dfc für jede Sättigungsstufe nach Formel (2); und
(7) Speichern der Kernsättigung und der berechneten Dispersionsdaten und Erstellen eines Sw-Dfc-Kreuzdiagramms durch Anpassen der gespeicherten Werte zum Erhalten einer Wassersättigungs-Dispersions-Beziehungsgleichung nach Formel (1).

## Revendications

1. Procédé de calcul de la saturation en eau d'une roche, **caractérisé en ce que** ladite saturation en eau de la roche est calculée par la formule (1) : Sw = aD_{fc} + b ;
dans la formule (1) :
Sw est la saturation en eau, en % ;
a est un coefficient, en (m·Hz)/F ;
b est un paramètre de régression, sans dimension ;
D_{fc} est la dispersion de la permittivité, en F/(m·Hz) ;
D_{fc} est calculée par la formule (2) ; ${D}_{fc} = \left|\frac{{dC}_{p}}{df}\right| = \left|{C}_{0}\right| {D}_{c} {f}^{- \left(Dc+1\right)}$ dans la formule (2) :
Cp est la permittivité, en F/m ;
D_{c} est l'indice de dispersion de la permittivité, sans dimension ;
f est la fréquence, en Hz ;
C₀ est le coefficient de permittivité, en F ;
Cp, C₀ et D_{c} sont mis en relation par la formule (3) : $Cp = {C}_{0} {f}^{- Dc}$ comprenant les étapes suivantes consistant à :
(1) mesurer le diamètre géométrique et le poids de la carotte ;
(2) mesurer la saturation en eau de la roche en déplaçant la saturation ;
(3a) saturer complètement la roche avec une solution saline d'une certaine concentration, avec la première saturation Sw = 100 % ;
(3b) mesurer le paramètre de permittivité de la roche dans une bande de fréquence (1 Hz à 10 kHz) dans le cas où Sw = 100 %, et enregistrer les données pertinentes ;
(4) changer la saturation en eau de la roche en déplaçant la saturation, réduire la saturation en eau de la roche, puis mesurer le paramètre de permittivité de la roche dans la même bande de fréquence après le déplacement ;
(5) établir un tracé croisé Cp-f par lissage afin d'obtenir une équation de la relation permittivité/indice de fréquence comme montré dans la formule (3) par lissage, dans lequel les paramètres de lissage dans la formule (3) sont C₀ et D_{c} ;
(6) calculer la dispersion D_{fc} pour chaque niveau de saturation en fonction de la formule (2) ; et
(7) enregistrer la saturation de la carotte et les données de dispersion calculées, et établir un tracé croisé Sw-D_{fc} par lissage desdites valeurs enregistrées afin d'obtenir une équation de la relation saturation en eau/dispersion en fonction de la formule (1).
